# EUROPÄISCHE PATENTSCHRIFT

(11) **EP 1 805 314 B1**
(45) Veröffentlichungstag und Bekanntmachung des Hinweises auf die Patenterteilung: **13.02.2008**
(21) Anmeldenummer: 05769677.5
(22) Anmeldetag: 28.07.2005
(51) Int. Cl.: C12P 7/24, C12P 7/40, C12P 7/62, C12P 17/02, C12P 41/00

(54) **VERFAHREN ZUR ENANTIOSELEKTIVEN ÖFFNUNG VON 3-SUBSTITUIERTEN OXETAN-2-ONEN UNTER VERWENDUNG DER LIPASEN VON CANDIDA ANTARCTICA ODER VON BURKHOLDERIA PLANTARII**
PROCESS FOR THE ENANTIOSELECTIVE OPENING OF 3-SUBSTITUTED OXETAN-2-ONES EMPLOYING LIPASES OF CANDIDA ANTARCTICA OR OF BURKHOLDERIA PLANTARRII
PROCEDE D'OUVERTURE ENANTIOSELECTIVE DES OXETAN-2-ONES 3-SUBSTITUEES EN UTILISANT LES LIPASES DE CANDIDA ANTARCTICA OU DE BURKHOLDERIA PLANTARRII

(30) Priorität: 02.08.2004 DE 102004037700; 06.08.2004 DE 102004038589
(43) Veröffentlichungstag der Anmeldung: 11.07.2007
(73) Patentinhaber: BASF AKTIENGESELLSCHAFT, 67056 Ludwigshafen (DE)
(72) Erfinder: HABICHER, Tilo, 67346 Speyer (DE); STÜRMER, Rainer, 67127 Rödersheim-Gronau (DE)
(86) Internationale Anmeldenummer: PCT/EP2005/008190
(87) Internationale Veröffentlichungsnummer: WO 2006/015727

(56) Entgegenhaltungen:
- SAKAI, N. ET AL.: "Lipase promoted asymmetric trans-esterification of 4-alkyl-, 3-alkyl- and 3,4-dialkyloxetan-2-ones with ring-opening" JOURNAL OF THE CHEMICAL SOCIETY. PERKIN TRANSACTIONS I, Nr. 1, 2000, Seiten 71-77, XP002372025 in der Anmeldung erwähnt
- ADAM, W. ET AL.: "Enzymatic preparation of optically active alpha-methylene beta-lactones by lipase-catalyzed kinetic resolution through asymmetric transesterification" TETRAHEDRON: ASYMMETRY, Bd. 8, Nr. 6, 27. März 1997 (1997-03-27), Seiten 833-836, XP004056867
- ITO, T. ET AL.: "Preparation and Use of Novel (S)-beta-Chlorodifluoromethyl-beta-propi olactone as a Chiral Fluorinated Building Block" TETRAHEDRON, Bd. 54, Nr. 21, 21. Mai 1998 (1998-05-21), Seiten 5523-5530, XP004118405

## Beschreibung

Die vorliegende Erfindung betrifft ein Verfahren zur Herstellung von im wesentlichen enantiomerenreinen 3-Hydroxycarbonsäuren bzw. -estern ausgehend von racemischen Oxetan-2-onen.

Optisch aktive 3-Hydroxycarbonsäuren und deren Ester sind gesuchte Zwischenprodukte zur Herstellung von Wirkstoffen für pharmazeutische Anwendungen.

### Stand der Technik:

Bekannt ist die katalytische Hydrierung von Ketonen und Ketoestern mit Ru-Diphosphin-komplexen (z.B. Burk et. al., J. Am. Chem. Soc 1995, 117, 4423; A. Mortreux et. al., Tetrahedron: Asymmetry, 7(2), 379-82, 1996; Noyori et. al. Angew. Chem., Int. Ed. Engl., 36(3), 285-288, 1997; WO 9713763 A1.

Bekannt ist ebenfalls die katalytische Transfer-Hydrierung von Ketonen mit Ameisensäure / Triethylamin-Komplex als Reduktionsmittel und Rutheniumkatalysatoren (P. Knochel et. al., Tetrahedron Lett., 37(45), 8165-8168, 1996; Sammakia et. al., J. Org. Chem., 62(18), 6104-6105, 1997 (Isopropanol als Reduktionsmittel)).

Gemeinsam ist diesen Methoden, dass sehr aufwendig herzustellende Katalysatoren und Liganden verwendet werden. Bei den Transferhydrierungen wird ferner nicht der billige Wasserstoff, sondern Isopropanol oder Ameisensäure/Tert. Amine verwendet. Letzteres erschwert die Aufarbeitung der Reaktion und führt zwangsläufig zum Anfallen von Aceton bzw. Kohlendioxid.

Ferner wird bei obigen Arbeiten in der Regel mit sehr grossen Katalysatormengen gearbeitet; dies macht die bisherigen Verfahren unwirtschaftlich.

Sakai et al. (J. Chem. Soc., Perkin Trans. 1, 2000, 71-77) beschreibt eine Lipasekatalysierte stereoselektive Umesterung von verschieden substituierten Oxetan-2-onen. Unter den beschriebenen Bedingungen werden jedoch noch keine voll zufriedenstellenden Umsetzungen hinsichtlich chemischer und optischer Ausbeute erhalten.

### Aufgabenstellung

Es bestand daher die Aufgabe, Verfahren zur enantioselektiven Öffnung von Oxetan-2-onen bereitzustellen, die die Nachteile der im Stand der Technik beschriebenen Verfahren beseitigen und insbesonders verbesserte chemische Ausbeuten und optische Reinheiten gewährleisten.

### Beschreibung der Erfindung

Die vorliegende Erfindung betrifft ein Verfahren zur Herstellung von im wesentlichen enantiomerenreinen 3-Hydroxycarbonsäuren bzw. -estern der allgemeinen Formel (III) indem man racemische Oxetan-2-one der allgemeinen Formel (I) mit Verbindungen R³-OH der allgemeinen Formel (II) in Gegenwart einer Lipase aus Candida antartica oder Burkholderia plantarii umsetzt und die erhaltenen Produkte der Formel (III) und (IV) voneinander trennt 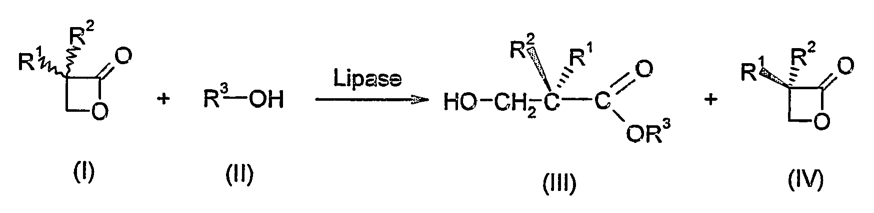 wobei die Reste folgende Bedeutung aufweisen:
R¹, R², R³ unabhängig voneinander H; C₁-C₁₀-substituiertes oder unsubstituiertes Alkyl, substituiertes oder unsubstituiertes Aryl oder Hetaryl, wobei R¹ und R² nicht gleichzeitig diesselbe Bedeutung besitzen dürfen. Der Begriff "C₁-C₁₀-substituiertes oder unsubstituiertes Alkyl" umfasst auch Cycloalkyle wie Cyclopentyl, Cyclohexyl, Cycloheptyl, Cyclopentylmethyl, Cyclopentylethyl, Cylcohexylmethyl, Cyclohexylethyl.

Die gezeigten Formelbilder (III) und (IV) stellen hier jeweils ein Enantiomer dar. Erfinderisch mitumfasst sind jedoch auch die Darstellung des jeweils anderen Enantiomeren (Antipoden) (III) und (IV). Die Bildung des gewünschten Enantiomeren kann durch Wahl der Lipase beeinflusst werden.

Racemische Oxetan-2-on der allgemeinen Formel (I) sind in der Literatur bekannt und können leicht durch bekannte Methoden - beispielsweise wie von Sakai et al. (J. Chem. Soc., Perkin Trans. 1, 2000, 71-77) beschrieben - erhalten werden.

Die Verbindungen (II) sind dem Fachmann bekannte Alkohole und Wasser. Je nachdem, ob die gewünschten 3-Hydroxycarbonsäuren (III) als freie Säuren oder direkt als Ester gewünscht werden, wählt man (II) als Wasser oder Alkohol.

Als Lipasen sind für das erfindungsgemässe Verfahren Lipasen aus den Organismen Candida antartica und Burkholderia plantarii geeignet. Mikroorganismen der Art Burkholderia plantarii werden inzwischen auch als Pseudomonas plantarii bezeichnet.

Solche Mikroorganismen sind bekannt und aus öffentlichen Stammsammlungen zugänglich, beispielsweise DSM No.9509, DSM No. 7128, DSM No. 9510, ATCC No. 51545, NCPPB No. 3676, ATCC No. 43733, ICMP No.9424, JCM No. 5492, LMG No. 9035.

Eine besonders gut geeignete Lipase kann aus dem Mikroorganismus DSM No. 8246 (hinterlegt am 28.04.1993) erhalten werden. Für die Herstellung der Lipase aus diesem Mikroorganismus wird auf die EP 1069183 insbesondere auf Beispiel 1 hingewiesen.

Mikroorganismen der Art Candida antarctica sind aus öffentlich zugänglichen Stammsammlungen erhältlich, beispielsweise DSM No. 70725. Die Mikroorganismen der Art Candida antarctica werden auch unter dem Namen Pseudozyma aphidis eingeordnet (beispielsweise DSM No. 70725, ATCC No. 32657, CBS No. 6821, NRRL No. Y-7954).

Eine besonders geeignete Lipase aus Candida antartica ist die kommerziell von NOVOZYMES erhältliche Lipase Novozym ^{®} 435.

Die Lipase aus Burkholderia plantarii bevorzugt bei der erfindungsgemässen Umsetzung die entgegengesetzte Stereochemie wie die Lipase aus Candida antarctica und ist demzufolge eine ideale Ergänzung des synthetischen Repertoires. Die genaue Stereochemie ist aus den Beispielen 1 und 2 ersichtlich.

Die Lipasen können für das erfindungsgemässe Verfahren sowohl als Rohextrakt als auch in unterschiedlich reinen Präparationen bis hin zu hochgereinigter Form eingesetzt werden. In einer bevorzugten Ausführungsform haben die Lipasen katalytische Aktivitäten von .0.1-1000., bevorzugt 10-400, besonders bevorzugt 20-200Units pro mg (gemessen als Tributyrin units).

Die Lipase Aktivität kann mit Hilfe bekannter Verfahren bestimmt werden (Gupta et al. Review: Lipase assays for conventional and molcular screening: an overview., Biotechnol. Appl. Biochem. (2003) 37, 63-71), beispielsweise dem titrimetrischen Tributyrin-Test.

Eine besonders bevorzugte Ausführungsform ist die Verwendung von an Träger gebundenen (immobilisierten) Lipasen. Solche Lipasen bzw. die Verfahren zu ihrer Immobilisierung sind beispielsweise aus EP 1069183 und den dort zitierten Dokumenten bekannt.

Die Reste R¹, R², und R³ in den Formeln (I) bis (IV) bedeuten Wasserstoff, C₁-C₁₀-substituiertes oder unsubstituiertes Alkyl, substituiertes oder unsubstituiertes Aryl oder Hetaryl. Unsubstituiertes Alkyl bedeutet hier insbesondere Methyl, Ethyl, n- und iso-Propyl, n-, iso-, tert. Butyl, geradkettiges und verzweigtes Pentyl, Hexyl, Heptyl, Octyl, Nonyl und Decyl sowie die verzweigten Alkyle beispielsweise Cyclobutan, Cyclopentan, Cyclohexan. Substituiertes Alkyl bedeutet hier ein Rest bei dem gegenüber dem entsprechenden unsubstituierten Alkylrest ein oder mehrere H-Atome durch andere Atome oder Molekülgruppen wie NH₂, N(Alkyl)H, N(Alkyl)₂, OH, O-Alkyl, SH, S-Alkyl, CN, NO₂, J; Cl, Br, F, Carbonyl, Carboxyl, Ester, Aryl oder Hetaryl ersetzt sind. Weiterhin sind von der Definition substituierte Alkyle auch ein- oder mehrfach ungesättigte Alkyle wie Alkene und Alkine mit umfasst.

Unsubstitutierte Aryle sind insbesondere Phenyl und Naphtyl, unsubstituierte Hetaryle sind solche aromatischen Verbindungen, bei denen mindestens ein C-Atom durch ein sogenanntes Heteroatom wie O, N, S, ersetzt ist. Bevorzugte Hetaryle sind Pyrryl, Furyl, Thiophenyl, Pyridyl, Pyrimidyl.

Die Reste R¹ und R² dürfen allerdings nicht gleichzeitig die gleiche Bedeutung besitzen, da sonst kein optisch aktives C-Atom entsteht und eine Auftrennung des Racemat (I) entfällt.

Das erfindungsgemässe Verfahren kann mit oder ohne Lösungsmittel ausgeführt werden. Bevorzugt wird jedoch ein Lösungsmittel verwendet, insbesondere aus der Gruppe der Alkylether oder es wird das Edukt (II) als zusätzlich auch als Lösungsmittel eingesetzt. Besonders bevorzugt wird Methyl-tert. Butylether und Diisopropylether als Lösungsmittel eingesetzt.

Eine Konkurrenzreaktion zur Ringöffnung des Oxetan-2-ons (I) mit dem Alkohol (II) ist die Ringöffnung von (I) durch (III) oder auch die Umesterung von (III) mit einem weiteren Molekül (III), wodurch eine "Dimerenbildung" von (III) erreicht wird. Um diese unerwünschte Konkurrenzreaktionen möglichst weitgehend zu unterdrücken, empfiehlt es sich mit einem Lösungsmittel, ggf. mit dem Alkohol (III) als Lösungsmittel zu arbeiten. Besonders bevorzugt wird ein Lösungsmittel in einer Menge von bis zu 25 Gew.-% bezogen auf (I) verwendet.

Da keine Lipase zu 100% stereoselektiv arbeitet, entsteht bei entsprechend langer Reaktionszeit immer auch ein gewisser Anteil des nicht-gewünschten Enantiomers als 3-Hydroxycarbonsäure bzw. -ester (III). Daher ist die für die Reaktion gewählte Reaktionszeit ein Kompromiss zwischen Reaktionsausbeute und optischer Reinheit der Produkte. Lange Reaktionszeiten führen in der Regel zu hohen Ausbeuten auf Kosten der optischen Reinheit, während kürzere Reaktionszeiten zu hohen optischen Reinheiten der Produkte führen, allerdings auf Kosten der Gesamtausbeute. Je nach Art der Reaktanden und der gewählten Bedingungen ist es deshalb ratsam in orientierenden Vorversuchen eine Reaktionskinetik aufzunehmen und daraus die optimale Reaktionszeit abzuleiten.

Die Reaktionszeit der enzymkatalysierten Reaktion hängt auch sehr stark von der gewählten Temperatur ab. Die Reaktion kann in einem weiten Temperaturbereich ausgeführt werden, bevorzugt bei solchen Temperaturen, bei denen die verwendete Lipase hinreichend aktiv ist. Bevorzugte Temperaturen sind zwischen 5 und 70, insbesondere zwischen 10 und 50°C.

Die Reaktion kann sowohl kontinuierlich als auch diskontinuierlich durchgeführt werden. Für eine Durchführung im technischen Massstab wird die kontinuierliche Synthese, insbesondere unter Verwendung einer geträgerten Lipase, empfohlen.

Nach erfolgter Umsetzung der Edukte (I) und (II) liegen die Produkte (III) und (IV) nebeneinander vor. Eine Trennung der Produkte (III) und (IV) gelingt mit üblichen Mitteln aufgrund der unterschiedlichen chemischen Struktur. Bevorzugt werden zur Trennung destillative Verfahren oder Extraktionsverfahren verwendet. Falls (III) als Säure vorliegt (R³=H), kann (III) bevorzugt in Form seines Alkali - oder Ammoniumsalzes von (IV) abgetrennt werden.

Das nicht gewünschte Enantiomer (IV) kann auch wieder nach Racemisierung in den Reaktionsansatz zurückgeführt werden. Es kann aber auch aus (IV) die entsprechende 3-Hydroxycarbonsäure bzw. -ester (III) gewonnen werden durch Hydrolyse unter Erhalt des optisch aktiven Zentrums.

Die vorliegende Erfindung kann auch zur Herstellung von im wesentlichen enantiomerenreinen Oxetan-2-onen der allgemeinen Formel (IV) verwendet werden.

Ein weiterer Gegenstand der Erfindung ist somit ein Verfahren zur Herstellung von im wesentlichen enantiomerenreinen Oxetan-2-onen der allgemeinen Formel (IV) indem man racemische Oxetan-2-one der allgemeinen Formel (I) mit Verbindungen R³-OH der allgemeinen Formel (II) in Gegenwart einer Lipase aus Candida antartica oder Burkholderia plantarii umsetzt und die erhaltenen Produkte der Formel (III) und (IV) voneinander trennt 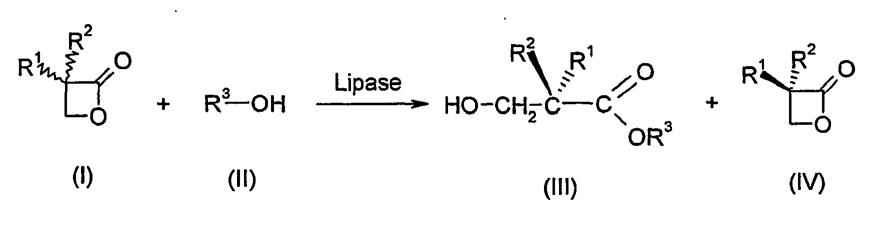 wobei die Reste folgende Bedeutung aufweisen:
R¹, R², R³ unabhängig voneinander H; C₁-C₁₀-substituiertes oder unsubstituiertes Alkyl, substituiertes oder unsubstituiertes Aryl oder Hetaryl, wobei R¹ und R² nicht gleichzeitig diesselbe Bedeutung besitzen dürfen. Der Begriff "C₁-C₁₀-substituiertes oder unsubstituiertes Alkyl" umfasst auch Cycloalkyle wie Cyclopentyl, Cyclohexyl, Cycloheptyl, Cyclopentylmethyl, Cyclopentylethyl, Cylcohexylmethyl, Cyclohexylethyl.

### Experimenteller Teil

### Beispiel 1 (Umsetzung mit Candida antarctica Lipase)

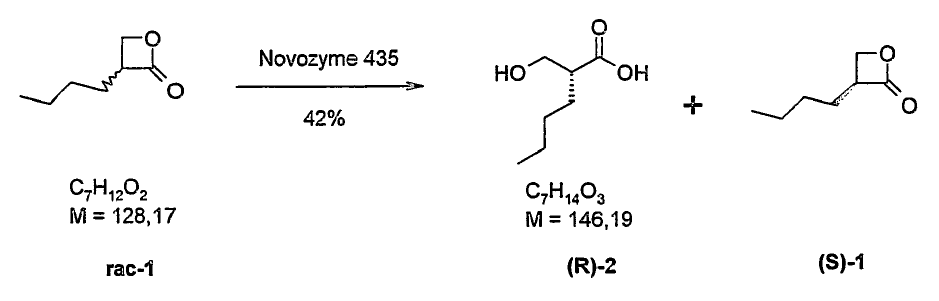

| | |
|---|---|
| Apparatur: | 4000ml BASF-Miniplant Rührgefäß, 3-Flügel-Metallpropellerrührer (d=10cm) mit 4 Strömungsbrechern, 164upm, Thermostat Innentemperatur geregelt |
| Ansatz: | |
| 3-Butyl-oxetan-2-on (6) | 121 g (0,95 mol) |
| Methyl-*tert.*-butylether | 1200 ml (Reaktion) |
| | 4300 ml (Extraktion) |
| Novozym 435 | 1,94g (1,6%) |
| VE-Wasser | 10,05 ml (0,56 mol) |
| NaHCO₃-Lsg. (10%) | 795 ml (0,95 mol) |
| Schwefelsäure (50%) | 111,3g (0,57 mol) |

### Durchführung:

| | |
|---|---|
| 121 g (0,95 mol) | rac-Lacton 6 werden in |
| 1200 ml | Methyl-*tert*.-butylether vorgelegt und mit |
| 1,94g (1,6%) | Novozym ® 435 und anschließend mit |
| 10,05 ml (0,56 mol) | VE-Wasser versetzt. |
| | 17h bei 25,0°C rühren. |
| | Filtration des Enzyms. |
| | Waschen der organischen Lösung mit |
| 795ml | NaHCO₃-Lösung (10%). pH 8,65 |
| | Phasentrennung |
| | Nachextraktion mit |
| 2x 800ml | Methyl-*tert*.-butylether |
| | Phasentrennung |
| | Alle 3 organischen Phasen vereinigen. (Lacton) |
| | Die wässrige Phase ansäuern mit H₂SO₄ (pH <3,0) und |
| | dreimal mit |
| (3x) 900ml | Methyl-*tert*.-butylether extrahieren. |
| | Alle 3 organischen Phasen vereinigen. (Säure) |
| | Trocknung über Natriumsulfat. Filtrieren. |
| | Das Lösungsmittel wird am Rotationsverdampfer entfernt. |

| | | |
|---|---|---|
| Ausbeute: | Säure = 58,2g (0,40 mol), 42% | chirale HPLC (GKA): >99%ee |
| | Lacton = 70,0g (0,55 mol), 58% | chirale GC (GVF-C): 80,03% |
| | Chiraler Umsatz = 44,7% | |

### Beispiel 2 (Umsetzung mit Burkholderia plantarii Lipase (DSM 8246))

Unter gleichen Bedingungen wie in Beispiel 1 -allerdings wurde eine Lipase aus Burkholderia plantarii anstatt Novozym® 435 verwendet (40 mg, 75 U/mg (Tributyrin-units) - wurden Produkte mit entgegengesetzter Stereochemie erhalten. Es wurden aus rac-**1** (2 g, 15,60 mmol) (S)-**2** (0,85 g; 5,6 mmol; 37%; 94ee) und (R)-**1** (1,08 g; 8,2 mmol; 54%; 95ee) erhalten.

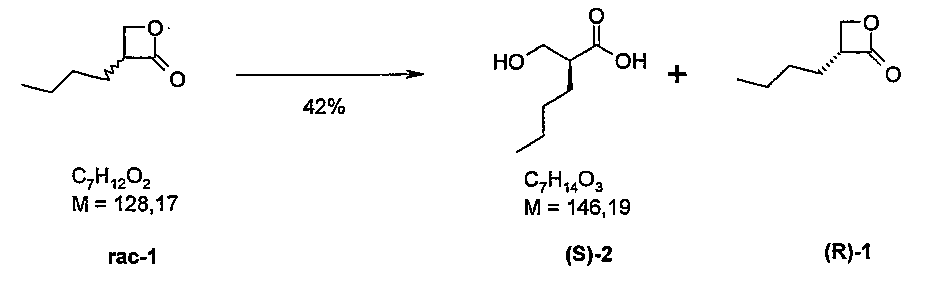

### Beispiel 3

### Umsetzung von verschieden substituierten Oxetan-2-onen mit Candida antarctica und Burkholderia plantarii Lipasen

Analog zu Beispiel 1 und 2 wurden die in der folgenden Tabelle genannten Substrate der allgemeinen Formel (I) umgesetzt und die entsprechenden Säuren der allgemeinen Formel (III) ("R- bzw. S-Säure") bzw. Oxetan-2-one der allgemeinen Formel (IV) ("R-bzw. S-Lacton") in der angegebenen Stereochemie erhalten.

| Enzym | R²=Me, R¹=H | R²=Et, R¹=H | R²=n-Pr, | R²=i-Pr, R¹=H |
|---|---|---|---|---|
| | | | R¹=H | |
| Novozym ^{®}435 | 98,5 % ee | 99 % ee | 99 % ee | 99 % ee |
| | R-säure | R-säure | R-säure | R-säure |
| | 99 % ee | 98 % ee | 98 % ee | 99 % ee |
| | S-lacton | S-lacton | S-lacton | S-lacton |
| Lipase BP (DSM 8246) | 98 % ee | 97.5 % ee | 99 % ee | 99 % ee |
| | S-säure | S-säure | S-säure | S-säure |
| | 98 % ee | 98 % ee | 97 % ee | 99 % ee |
| | R-lacton | R-lacton | R-lacton | R-lacton |

## Patentansprüche

1. Verfahren zur Herstellung von im wesentlichen enantiomerenreinen 3-Hydroxycarbonsäuren bzw. -estern der allgemeinen Formel (III), indem man racemische Oxetan-2-one der allgemeinen Formel (I) mit Verbindungen R3-OH der allgemeinen Formel (II) in Gegenwart einer Lipase aus Candida antartica oder Burkholderia plantarii umsetzt und die erhaltenen Produkte der Formel (III) und (IV) voneinander trennt 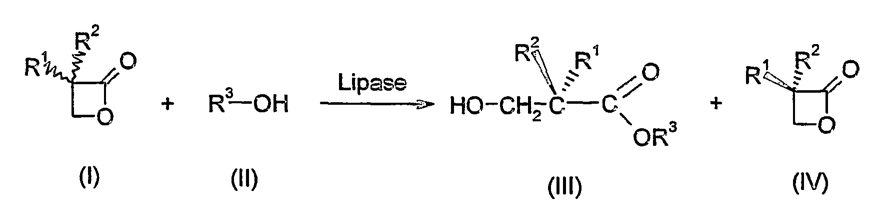 wobei die Reste folgende Bedeutung aufweisen:
R¹, R², R³ unabhängig voneinander H; C₁-C₁₀-substituiertes oder unsubstituiertes Alkyl, substituiertes oder unsubstituiertes Aryl oder Hetaryl, wobei R¹ und R² nicht gleichzeitig diesselbe Bedeutung besitzen dürfen.

2. Verfahren zur Herstellung von im wesentlichen enantiomerenreinen Oxetan-2-onen der allgemeinen Formel (IV), indem man racemische Oxetan-2-one der allgemeinen Formel (I) mit Verbindungen R³-OH der allgemeinen Formel (II) in Gegenwart einer Lipase aus Candida antartica oder Burkholderia plantarii umsetzt und die erhaltenen Produkte der Formel (III) und (IV) voneinander trennt 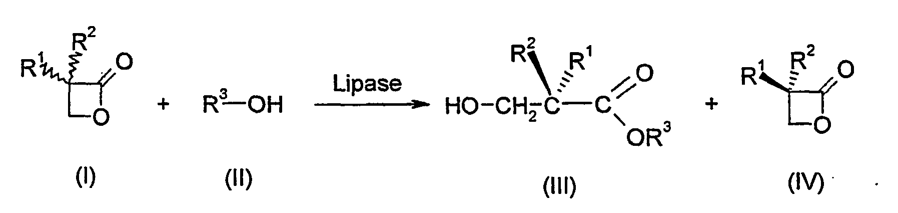 wobei die Reste folgende Bedeutung aufweisen:
R¹, R², R³ unabhängig voneinander H; C₁-C₁₀-substituiertes oder unsubstituiertes Alkyl; substituiertes oder unsubstituiertes Aryl oder Hetaryl, wobei R¹ und R² nicht gleichzeitig diesselbe Bedeutung besitzen dürfen.

3. Verfahren nach Anspruch 1 oder 2, **dadurch gekennzeichnet, dass** R¹ =H und R² = n-Butyl, Methyl, Ethyl, iso-propyl, Phenyl bedeutet.

4. Verfahren nach Anspruch 1 oder 2, **dadurch gekennzeichnet, dass** als Lösungsmittel für die Umsetzung Methyl-tert.Butylether verwendet wird.

5. Verfahren nach Anspruch 1 oder 2, **dadurch gekennzeichnet, dass** eine an Träger gebundene Lipase verwendet wird.

6. Verfahren nach Anspruch 1 oder 2, **dadurch gekennzeichnet, dass** die Reaktion kontinuierlich durchgeführt wird.

7. Verfahren nach Anspruch 1 oder 2, **dadurch gekennzeichnet, dass** die Trennung der Produkte (III) und (IV) durch Destillation oder Extraktion bzw. einer Kombination aus beiden Methoden geschieht.

## Claims

1. A method for preparing substantially enantiopure 3-hydroxy carboxylic acids or esters of the general formula (III), by reacting racemic oxetan-2-ones of the general formula (I) with compounds R³-OH of the general formula (II) in the presence of a lipase from Candida antarctica or Burkholderia plantarii, and separating the resulting products of the formula (III) and (IV) from one another 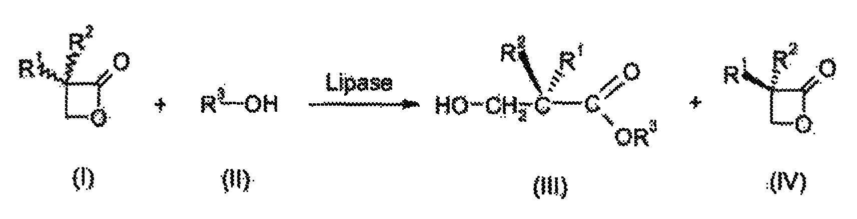 where the radicals have the following meaning:
R¹, R², R³ independently of one another H; C₁-C₁₀-substituted or unsubstituted alkyl, substituted or unsubstituted aryl or hetaryl, where R¹ and R² may not simultaneously have the same meaning.

2. A method for preparing substantially enantiopure oxetan-2-ones of the general formula (IV), by reacting racemic oxetan-2-ones of the general formula (I) with compounds R³-OH of the general formula (II) in the presence of a lipase from Candida antarctica or Burkholderia plantarii, and separating the resulting products of the formula (III) and (IV) from one another 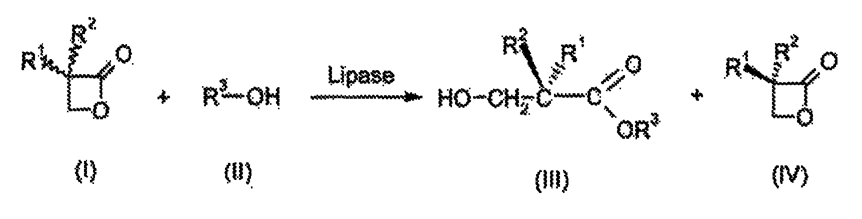 where the radicals have the following meaning;
R¹, R², R³ independently of one another H; C₁-C₁₀-substituted or unsubstituted alkyl, substituted or unsubstituted aryl or hetaryl, where R¹ and R² may not simultaneously have the same meaning.

3. The method according to claim 1 or 2, wherein R¹ is H and R² is n-butyl, methyl, ethyl, isopropyl, phenyl.

4. The method according to claim 1 or 2, wherein methyl tert-butyl ether is used as solvent for the reaction.

5. The method according to claim 1 or 2, wherein a carrier-bound lipase is used.

6. The method according to claim 1 or 2, wherein the reaction is carried out continuously.

7. The method according to claim 1 or 2, wherein the separation of the products (III) and (IV) takes place by distillation or extraction or a combination of both methods.

## Revendications

1. Procédé de préparation d'acides ou d'esters 3-hydroxycarboxyliques essentiellement exempts d'énantiomères de formule générale (III) en mettant en réaction des oxétan-2-ones racémiques de formule générale (I) avec des composés R³-OH de formule générale (II) en présence d'une lipase de Candida antarctica ou de Burkholderia plantarii et en séparant les produits obtenus de formule (III) et (IV) l'un de l'autre : 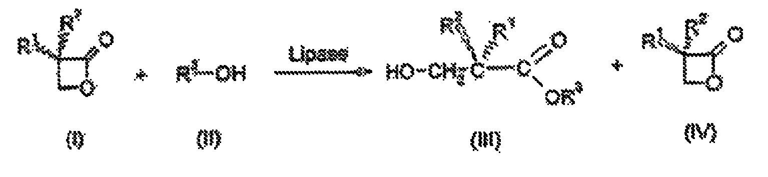 les radicaux présentant la signification suivante :
R¹, R² et R³ désignent, indépendamment les uns des autres, H; un radical alkyle non substitué ou substitué par un radical C₁-C₁₀, un radical aryle ou hétaryle substitué ou non substitué, R¹ et R² ne devant pas posséder simultanément la même signification.

2. Procédé de préparation d'oxétan-2-ones essentiellement exemptes d'énantiomères de formule générale (IV) en mettant en réaction des oxétan-2-ones racémiques de formule générale (I) avec des composés R³-OH de formule générale (II) en présence d'une lipase de Candida antarctica ou de Burkholderia plantarii et en séparant les produits obtenus de formule (III) et (IV) l'un de l'autre : 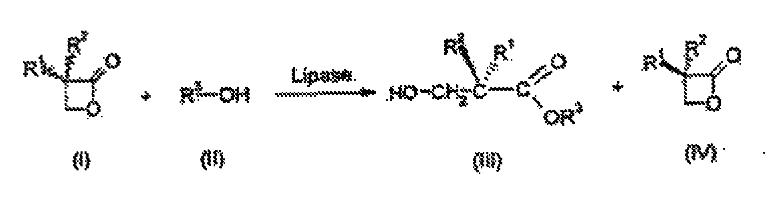 les radicaux présentant la signification suivante :
R¹, R² et R³ désignent, indépendamment les uns des autres, H; un radical alkyle non substitué ou substitué par un radical C₁-C₁₀, un radical aryle ou hétaryle substitué ou non substitué, R¹ et R² ne devant pas posséder simultanément la même signification.

3. Procédé selon l'une des revendications 1 ou 2, **caractérisé en ce que** R¹ = H et R² = n-butyle, méthyle, éthyle, isopropyle ou phényle.

4. Procédé selon l'une des revendications 1 ou 2, **caractérisé en ce que** du méthyl-tert.-butyléther est utilisé comme solvant pour la réaction.

5. Procédé selon l'une des revendications 1 ou 2, **caractérisé en ce qu'**une lipase liée sur un support est utilisée.

6. Procédé selon l'une des revendications 1 ou 2, **caractérisé en ce que** la réaction se déroule en continu.

7. Procédé selon l'une des revendications 1 ou 2, **caractérisé en ce que** la séparation des produits (III) et (IV) est effectuée par distillation ou extraction ou une combinaison des deux méthodes.
